(19) Europäisches Patentamt / European Patent Office / Office européen des brevets

(11) **EP 2 751 268 B1**

(12) **EUROPEAN PATENT SPECIFICATION**

(45) Date of publication and mention of the grant of the patent:
**06.04.2016 Bulletin 2016/14**

(21) Application number: **12745471.8**

(22) Date of filing: **02.08.2012**

(51) Int Cl.:
*C12N 15/10* [(2006.01)]   *C12Q 1/68* [(2006.01)]
*G01N 33/68* [(2006.01)]   *C12N 1/06* [(2006.01)]

(86) International application number:
**PCT/EP2012/065178**

(87) International publication number:
**WO 2013/029919 (07.03.2013 Gazette 2013/10)**

(54) **METHOD FOR THE ISOLATION OF GENOMIC DNA, RNA, PROTEINS AND METABOLITES FROM A SINGLE BIOLOGICAL SAMPLE**

VERFAHREN ZUR ISOLIERUNG VON GENOMISCHER DNA, RNA, PROTEINEN UND METABOLITEN AUS EINER BIOLOGISCHEN PROBE

MÉTHODE POUR ISOLER L'ADN, L'ARN, LES PROTÉINES GÉNOMIQUES ET LES MÉTABOLITES D'UN SEUL ÉCHANTILLON BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2011 LU 91864**

(43) Date of publication of application:
**09.07.2014 Bulletin 2014/28**

(73) Proprietors:
• **Université du Luxembourg**
 **1511 Luxembourg (LU)**
• **Luxembourg Institute of Science and Technology (LIST)**
 **4362 Esch-sur-Alzette (LU)**

(72) Inventors:
• **WILMES, Paul**
 **3502 Dudelange (LU)**
• **ROUME, Hugo**
 **1471 Luxembourg (LU)**
• **HILLER, Karsten**
 **66663 Besseringen (DE)**
• **CORDES, Thekla**
 **1630 Luxembourg (LU)**

(74) Representative: **Lecomte & Partners**
 **P.O. Box 1623**
 **1016 Luxembourg (LU)**

(56) References cited:
 **US-A1- 2004 224 344**

• **WOLFRAM WECKWERTH ET AL: "Process for the integrated extraction, identification and quantification of metabolites, proteins and RNA to reveal their co-regulation in biochemical networks", PROTEOMICS, vol. 4, no. 1, 1 January 2004 (2004-01-01) , pages 78-83, XP055013545, ISSN: 1615-9853, DOI: 10.1002/pmic.200200500 cited in the application**
• **Norgen Biotek Corp.: "All-in-One Purification Kit Product #24200", , 2008, pages 1-26, XP002664869, Retrieved from the Internet: URL:http://www.norgenbiotek.com/product_re sources/allinone_purification_kit_total_rn a_microrna_total_proteins_and_genomic_dna_ allinone_purification_kit__protocol_24200_ 110.pdf [retrieved on 2011-12-01]**
• **GROSS: "Tissue fractionation by hydrostatic pressure cycling technology: the unified sample preparation technique for systems biology studies", JOURNAL OF BIOMOLECULAR TECHNIQUES, vol. 19, no. 3, 1 January 2008 (2008-01-01), page 189, XP055013562, ISSN: 1524-0215 cited in the application**
• **SOROTH CHEY ET AL: "Improved method for simultaneous isolation of proteins and nucleic acids", ANALYTICAL BIOCHEMISTRY, vol. 411, no. 1, 1 April 2011 (2011-04-01) , pages 164-166, XP055013548, ISSN: 0003-2697, DOI: 10.1016/j.ab.2010.11.020**

**(Cont. next page)**

- SHIBKO S ET AL: "A method for sequential quantitative separation and determination of protein, RNA, DNA, lipid, and glycogen from a single rat liver homogenate or from a subcellular fraction", ANALYTICAL BIOCHEMISTRY, ACADEMIC PRESS INC, NEW YORK, vol. 19, no. 3, 1 June 1967 (1967-06-01), pages 514-528, XP024827657, ISSN: 0003-2697, DOI: 10.1016/0003-2697(67)90242-4 [retrieved on 1967-06-01]

**Description**

The invention relates to a method for the isolation of genomic DNA, RNA, proteins, and polar and non-polar metabolites from a single biological sample.

Microbial communities are vital for the functioning of all eco-systems. At present, the vast majority of microorganisms are considered unculturable, and their roles in natural systems are largely unknown. The direct application of high-resolution molecular biology methodologies ("omics") is facilitating the study of the genetic and functional potential within natural microbial communities as well as in other biological systems including humans.

A major challenge in the emerging field of molecular (eco-)systems biology is to comprehensively characterise the extensive complexity that exists within microbial consortia. To understand biology at the system level, the structure and dynamics of cellular and organismal function must be examined in an integrated way, rather than only considering the individual characteristics of isolated parts of cells or organisms. An important consideration is the need to obtain comprehensive and representative biomolecular fractions of DNA, RNA, proteins and metabolites which can then be analysed using dedicated instrumentation. Due to their interconnectivity, the integration of the resulting high-resolution systems-level molecular data, obtained through genomics (high-throughput analyses of genomic DNA), transcriptomics (high-throughput analyses of RNA), proteomics (high-throughput analyses of proteins) and metabolomics (high-throughput analyses of metabolites) will enable systems-level overviews of community- and population-wide processes. This will in particular facilitate a more complete picture of microbial community composition, interaction, and evolution which in turn could inform future strategies that will allow us to steer microbial communities towards particular end points, which in turn may be of pronounced biotechnological interest.

Powerful and sensitive methods are available for the analysis of nucleic acids (DNA and RNA), proteins and small molecules. However, molecular (eco-)systems biology studies based on these analyses are facing major bottlenecks arising from the dynamic nature and extensive heterogeneity of microbial consortia in space-time dimensions. Multiple mutually exclusive sample preparations, which are often required to extract distinct classes of molecules from cell or tissues, is inconsistent with the need for comprehensive integration of systems-level data. Consequently, understanding a system's structure and dynamics requires integrated metabolomic, genomic, transcriptomic, and proteomic analyses, which demands the concomitant and comprehensive isolation of metabolites, nucleic acids and proteins from the same sample.

Current methodologies for concomitant isolation of DNA, RNA and proteins are primarily based on monophasic mixtures of water, phenol and guanidine isothiocyanate commercially available as TRIzol® or TRI Reagent®. The guanidine isothiocyanate is used simultaneously to lyse the cells, denature and inactivate proteins, including RNases, and separate rRNA from ribosomes during the initial RNA isolation step. Poor solubility solvents such as phenol and water maintained at a low pH, are used with chloroform for partitioning the RNA by dissolution and centrifugation, DNA and proteins fractions separate from the homogenate in aqueous, inter- and organic phases. Several improved methods for isolation and solubilisation of proteins after TRIzol® extraction of RNA and DNA have been described in the following articles:

- Hummon AB, Lim SR, Difilippantonio MJ, Ried T. (2007). Isolation and solubilization of proteins after TRIzol extraction of RNA and DNA from patient material following prolonged storage. Biotechniques 4:467-70, 472.
- Chey S, Claus C, Liebert UG. (2011). Improved method for simultaneous isolation of proteins and nucleic acids. Analytical Biochemistry 1:164-166.
- Xiong J, Yang Q, Kang J, Sun Y, Zhang T, Margaret G et al. (2011). Simultaneous isolation of DNA, RNA, and protein from Medicago truncatula L. ELECTROPHORESIS 2:321-330.

US7488579 also discloses a method for simultaneously extracting DNA, RNA and protein from the same biological sample involving phase separation. DNA and RNA can be extracted from an upper aqueous phase as separated DNA and RNA. Proteins can be extracted from a lower organic phase. Importantly, this protocol does not disclose how to further extract metabolites from the same sample.

A simultaneous extraction of metabolites, proteins and RNA was performed, on plant material, for revealing a co-regulation in biochemical network and described in "Weckwerth W, Wenzel K, Fiehn O. (2004): Process for the integrated extraction, identification and quantification of metabolites, proteins and RNA to reveal their co-regulation in biochemical networks. PROTEOMICS 1:78-83."

This process was based first on sample homogenization step by cryo-milling in liquid nitrogen. A solvent mixture of methanol, chloroform and water (2,5/1/1, volume to volume) was used then for precipitating proteins and nucleic acids and to disassociate metabolites from membrane and cell wall components. Finally proteins and RNA, contained in a pellet, were extracted by methanol/chloroform and phenol buffer.

However, the lysis step, which is crucial to determine the quality and the quantity of biomolecular fractions isolated, results in a loss of genomic DNA and protein material during metabolite extraction if not carried in a comprehensive way.

Furthermore, excessive mechanical lysis through cryomilling required for comprehensive lysis as per Weckwerth *et al.,* will result in a low quality and low molecular weight DNA which will be useless for genomics.

US2005/0106604 discloses a phase isolation process for biomolecules in which, after centrifugation, metabolites such as lipids are found in the upper phase, proteins precipitate in the middle phase, plasmid DNA, viral nucleic acid, mitochondrial DNA are in the lower phase, RNA precipitates in the lower phase, genomic DNA precipitates in the lower phase or in the middle phase. This process allows a separation of non-polar metabolites, DNA, RNA and proteins from the same sample, but does not allow an isolation of polar metabolites. In addition, small RNA is not separated from total RNA. Genomic DNA precipitates in both the middle phase and lower phase and a further separation is then required which is not described.

From prior art, methods for isolation of biomolecules using chromatographic spin columns have been reported. These methods rely on binding by adsorption of nucleic acids to solid phases such silica or glass particles, depending on the pH and the salt content of the buffer(s) used. The solid phase is washed and the biomolecules of interest specifically elute thanks to a specific pH and salt buffer. A first application of spin column-based methods was developed for a combined extraction of RNA and proteins and described in the following articles:

- Morse SM, Shaw G, Larner SF. (2006). Concurrent mRNA and protein extraction from the same experimental sample using a commercially available column-based RNA preparation kit. Biotechniques 1:54, 56, 58-
- Tolosa JM, Schjenken JE, Civiti TD, Clifton VL, Smith R. (2007). Column-based method to simultaneously extract DNA, RNA, and proteins from the same sample. Biotechniques 6:799-804.

WO2009/070558 discloses also a method for isolating genomic DNA, RNA and proteins. After lysis of cells, DNA is bound on a first mineral support while the flow-through contains both unbound total RNA and proteins. Then RNA is bound to a second mineral support. The proteins are contained within the second flow-through. Small RNA can be isolated from the total RNA fraction. The method involves the use of chaotropic salt in the lysis solution which is essential to inhibit RNases and proteases, but which is also known to the skilled man to alter the lipid fraction. Therefore this method is not compatible with the simultaneous isolation of the non-polar metabolite fraction.

A simultaneous isolation of DNA, RNA, proteins and lipids from cells and tissues based on physical disruption of the cellular material by hydrostatic pressure and the development of a new ProteoSolve-SB kit developed for systems biology studies has been described in "Gross V, Carlson G, Kwan AT, Smejkal G, Freeman E, Ivanov AR et al. (2008). Tissue fractionation by hydrostatic pressure cycling technology: the unified sample preparation technique for systems biology studies. J Biomol Tech 3:189-199." One of the claimed advantages of this method is to avoid labor-intensive and inconsistent tissue disruption steps like sonication and grinding in liquid nitrogen. However, this protocol does not disclose how to further isolate polar metabolites nor does it fractionate the RNA fraction into large and small RNA fractions. There is a definite need for an efficient and accurate protocol able to separate all known biomolecular fractions such as genomic DNA, large and small RNA, proteins, and polar and non-polar metabolites from the same sample in order to understand a biological system's structure and dynamics. Such need is also felt when working with samples that are precious or unique like biopsy tissue or samples that are difficult to replicate, such as small cell populations or indeed mixed microbial communities that are highly dynamic in terms of composition and function.

The objective of the invention is to overcome the prior art's disadvantages and to provide the skilled man with a new and universal extraction protocol able to concomitantly isolate cellular polar and non-polar metabolites, genomic DNA, large RNA, small RNA and proteins from unique samples.

A first aspect of the invention is a method for the separation and purification of cellular components from a single biological sample, the cellular components comprising polar and non-polar metabolites, genomic DNA, RNA and proteins, the method encompassing the following steps:

a) performing a mechanical lysis and homogenization of the single biological sample such that a part of the cells are lysed, the mechanical lysis being halted when 30 to 60 % of cells have been lysed;
b) performing a metabolite extraction on the homogenized single biological sample from step (a) by addition of a phase separation solution, homogenization by oscillation and centrifugation to form an upper phase, an interphase pellet and a lower phase; such that polar metabolites are in the upper phase, genomic DNA, RNA and proteins and the remaining cells not lysed by the mechanical lysis are in the interphase pellet, and non-polar metabolites are in the lower phase;
c) collecting separately the upper phase, the lower phase and the interphase pellet;
d) adding a lysis solution to the collected interphase pellet to perform a chemical lysis or a combined mechanical and chemical lysis in order to obtain a lysate;
e) performing a sequential isolation of genomic DNA, RNA and proteins on the lysate.

Preferably, the mechanical lysis of step (a) is halted when about 50 % of cells have been lysed.

With preference, the mechanical lysis of step (a) is performed under conditions to preserve RNA. With preference, the mechanical lysis of step (a) further comprises the addition of a solution preserving RNA, for example the addition of RNAlater. Preferably, the mechanical lysis of step (a) is a cryo-milling step. With preference the cryo-milling step is performed at a temperature between about -60°C and-196°C.

Preferably, the cryo-milling step is performed in an oscillating mill at a frequency of 20 to 40 Hz, preferably 30 Hz. Preferably the cryo-milling step is performed during about 1 to 3 min, preferably during about 2 min, and preferably during substantially 2 min.

Preferably, the phase separation solution of step (b) comprises a mixture of methanol and chloroform and water in the proportion of 1 volume of methanol, 1 volume of water and two volumes of chloroform.

Preferably, the addition of the phase separation solution of step (b) and the homogenizing of the sample is performed at a temperature below 0°C.

Preferably, in step (d) the lysis solution comprises Tris-EDTA and a lysis buffer. With preference the lysis buffer has Tris-HCl, EDTA, EGTA, SDS, deoxycholate or any combination thereof.

Preferably, in step (d) $\beta$-mercaptoethanol is further added to the interphase to preserve RNA integrity.

Preferably, the biological sample is obtained with the steps of:

- collecting a sample and snap-freezing said sample directly after collection in liquid nitrogen, with preference at a temperature of - 196 °C;
- thawing the sample to a temperature comprised between 0°C and 4°C;
- centrifuging to form a lower phase comprising biomass, and an upper phase comprising supernatant;
- collecting said biomass and freezing said biomass with preference at a temperature between -60°C and -196°C, with preference at a temperature of-196°C;
- using the frozen biomass as the single biological sample starting material for step (a).

Preferably, the supernatant is collected and submitted to a metabolite extraction in order to extract extracellular metabolites.

Preferably, the metabolite extraction on the supernatant is performed with addition of a phase separation solution, homogeniziation and centrifugation of the mixture comprising the supernatant and the phase separation solution to form an upper phase, an interphase pellet and a lower phase; such that polar metabolites are in the upper phase, and non-polar metabolites are in the lower phase, with preference the phase separation solution consists of a mixture of methanol and chloroform and water in the proportion of 1 volume of methanol, 1 volume of supernatant and two volumes of chloroform.

Preferably, the sequential isolation of genomic DNA, RNA and proteins of step (e) comprises a step of isolation of small RNA from the single biological sample. Preferably said step of isolation of small RNA is performed in fractionating the total RNA isolated in a small RNA fraction and a large RNA fraction.

Preferably, the sequential isolation of genomic DNA, RNA and proteins of step (e) is carried out using chromatographic spin-columns.

Preferably, the sequential isolation of genomic DNA, RNA and proteins of step (e) further comprises the steps of

(e-1) Mixing lysate with dipolar atropic solvent or with polar tropic solvent such as ethanol to obtain a solution,
(e-2) Applying the solution of step (e-1) to a first chromatographic spin-column under conditions for genomic DNA, large RNA and part of the proteins to bind, and for obtaining a flowthrough;
(e-3) Collecting the flowthrough which contains small RNA and a part of the proteins;
(e-4) Applying the flowthrough of step (e-3) to a second chromatographic spin-column under conditions for small RNA to bind and for obtaining a flowthrough ; (e-5) Eluting the small RNA from the second chromatic spin-column;
(e-6) Eluting sequentially genomic DNA and large RNA from the first chromatographic spin-column;
(e-6) Collecting the flowthrough of step (e-4) and adjusting the pH with preference to pH 3,
(e-7) Applying the pH adjusted flowthrough of step (e-4) to the first chromatographic spin-column;
(e-8) Eluting proteins from the first chromatographic spin-column.

Another object of the disclosure is a kit which comprises consumables and instructions for the separation and purification of cellular components including polar and non-polar metabolites, genomic DNA, RNA and proteins from a single biological sample according to the method of the invention, the kit further comprising a phase separation solution, a lysis solution, two chromatographic spin-columns, wash and elution solutions for the genomic DNA, wash and elution solutions for total RNA fraction and/or for small RNA fraction and/or for large RNA fraction and with preference wash and elution solutions for proteins.

As it is understandable from the above definition, the invention provides a new and universal method for the concomitant extraction of total, polar and non-polar, metabolites, large and small RNA, genomic DNA and proteins from mixed microbial communities from large diversity of environmental or human-derived mixed microbial community samples. Comparative analysis and quality assessment revealed that the biomolecular fractions extracted by this method showed comparable yields but improved quality compared to widely used reference methods. Thus, this new method allows unique systems biology studies where correlation and biomolecular network modeling among metabolomic, transcriptomic, genomic and proteomic data were previously considered distorted or/and not adequate due to sample heterogeneity and system dynamics.

The present method lays the foundation to carry out comprehensive systems-level molecular surveys on a range of different microbial communities that may be of pronounced biotechnological and human health interest. The method according to the invention may further be applicable to biomedical samples such as tumor biopsies, whole blood, serum, plasma, etc., as well as, to cell cultures and plant material.

According to a second aspect, the invention is remarkable in that two steps of lysis of the cells are performed sequentially. The lysis steps are performed before and after the metabolite isolation. The first lysis step is mechanical whereas the second is chemical or both mechanical and chemical. Preferably, a combined mechanical and chemical lysis step is performed as second lysis step. The combined mechanical and chemical lysis step is performed for example by bead-beating the sample after addition of a lysis solution comprising a chaotropic agent.

The initial mechanical step is preferably a cryogenic grinding step (cryo-milling) which results in homogenization of the sample and partial cell lysis prior to metabolite extraction. This initial lysis step is performed in order to be incomplete, i.e. in order to lyse at least 30 % of the cells and not more than 60 %. The inventors demonstrate that this cryo-milling pretreatment does not affect the quality and the quantity of biomolecular fractions isolated, in particular the integrity of the DNA and RNA fractions. With preference the lysis step is halted when lysis about 50 % of the cells are lysed.

To conduct the first lysis in order to be partial presents several advantages. Firstly, the mechanical nature of the first lysis step does not involve chemical products such as chaotropic agents that may affect non-polar metabolites. Secondly, to halt the mechanical step before the lysis of the cells is complete avoid excessive milling and helps to preserve high quality and high molecular weight DNA, so the later isolated DNA is usable for genomic analyses. Thirdly, the partial nature of the lysis step allows obtaining a representative protein fraction by preserving a part of the cells intact, independent of their morphology or their identity, during the metabolite extraction. Indeed some proteins of the lysed cells that are released with the first mechanical step and which show hydrophobic properties, may dissolve in the non-polar solvent used during the metabolite extraction and be lost for proteomics. To preserve intact a significant fraction of the cells during the metabolite extraction step, and to lyse them after the metabolite extraction, aids in obtaining a protein fraction for proteomics that includes such hydrophobic proteins. Fourthly, to perform the first mechanical lysis step at very low temperature helps in preserving the respective biomolecules as a molecular snap-shot of the time of sampling.

In an embodiment, the mechanical lysis step is sonication or any known mechanical lysis step, and is performed under conditions to preserve RNA, for example by addition of a specific solution known to preserve RNA such as RNAlater.

The second lysis step is performed at room temperature and after the metabolite extraction and involves the use of a chaotropic reagent. It has to be noted that the chaotropic reagent is reserved until after metabolite extraction and so does not affect the metabolomic studies by altering non-polar metabolites. Indeed, in the second lysis step the biological sample is lysed in an aqueous lysis buffer system containing a chaotropic agent and/or other salts added to the sample. The used chaotropic agents disrupt the intermolecular forces between water molecules, allowing proteins, DNA and RNA to dissolve more easily. Importantly, the primary structure of a protein or of a nucleic acid is left intact while other structures such as secondary, tertiary or quaternary structures are altered. Exemplary chaotropic agents include, but are not limited to: Guanidine Hydrochloride, Guanidium Thiocyanate, Sodium Thiocyanate, Sodium Iodide, Sodium Perchlorate, Lithium Perchlorate and Urea. After the chemical lysis step in addition to the solvent mixing step at least 80 % and preferably at least 90 % of the cells are lysed.

According to a third aspect of the invention, the method also allows the isolation of both extra- and intra-cellular polar and non-polar metabolites. For example when the collected sample is a lipid accumulating organism enriched sample, the collected sample is first centrifuged in order to separate the biomass from a supernatant and the metabolite extraction is performed separately on supernatant (i.e. extracellular fraction) and biomass (i.e. intracellular fraction). The inventors show that intra- and extracellular metabolomes are distinct.

Pronounced variability is typically introduced into high-resolution omic experiments by replicate sampling or sample splitting before the dedicated isolation of the individual biomolecular fractions. It follows that such approaches create artefacts due to unbalanced component distribution and may results in conflicting results. Because of this disparity, multiple mutually exclusive biomolecular extractions will not allow the systemic biologist to comprehensively integrate high-resolution omic data following specialised analyses and, thus, such experiments will not allow meaningful reconstruction of complex biomolecular networks. A sequential biomolecular extraction protocol on a single sample should circumvent as much as possible this current bias.

<u>Definitions</u>

As used herein "cryo-milling" is equivalent to cryogenic grinding, freezer milling and/or freezer grinding and refers to the act of cooling or chilling a material and then reducing it into a small particle size.

As used herein "partial lysis" refers to a lysis process of cells conducted in order to be incomplete such that a significant fraction of the cells is not lysed.

As used herein "small RNA" refers to RNA below 200 nucleotides such as micro RNA and other RNA, e.g. tRNA.

As used herein "large RNA" refers to RNA above 200 nucleotides sucha as mRNA and rRNA.

As used herein "total RNA" refers to a mixture of both small RNA (<200 nt) and large RNA (>200 nt).

As used herein "large RNA fraction" refers to a RNA fraction comprising mainly large RNA.

As used herein "phase separation solution" is a mixture comprising polar solvents and non-polar solvents.

As used herein "phase separation" is a process by which a single phase separates into two or more new phases, the new phases being liquid or solid.

As used herein "metabolites" refers to any intermediate or product resulting from metabolism, i.e. from physical and chemical processes involved in the maintenance and reproduction of life in which nutrients are broken down to generate energy and simpler molecules which themselves may be used to form more complex molecules.

As used herein "polar metabolites" refers to all hydrophilic metabolites showing a capacity to interact with polar solvents, in particular with water or with other polar groups, such as sugars, amino acids, organics acid, etc.

As used herein "non-polar metabolites" refers to all hydrophobic metabolites having a tendency to dissolve in non-polar solvents, such as lipophilic compounds, lipids, waxes, chlorophyll, etc.

As used herein "indiscriminate cell type" means that all cells are concerned independent of e.g. their morphology, identity, etc.

The invention will now be described by way of examples with reference to the accompanying figures in which:

- Figure 1 shows a flowchart of the method according to the invention;
- Figure 2 shows a lysis efficiency chart;
- Figure 3 shows total ion chromatogram (TIC) obtained by gas chromatography coupled to mass spectrometry;
- Figure 4 shows electropherograms and associated gels of RNA fractions isolated using the method pertaining to the invention;
- Figure 5 shows agarose gel electrophoresis of genomic DNA fraction;
- Figure 6 shows SDS-PAGE gel electrophoresis of proteins fractions;
- Figure 7 shows comparative summary of yields obtained for each fraction of different methods;
- Figure 8 shows the method of the invention quality assessment;
- Figure 9 shows principal component analysis diagram of polar and non-polar metabolites obtained from intra- and extra-cellular sludge biomass;
- Figure 10 shows a diagram of Sorensen's similarity matrix obtained from intracellular polar metabolomic data;
- Figure 11 shows diagram of Bray-Curtis dissimilarity matrix obtained from intra-cellular polar metabolomic data.

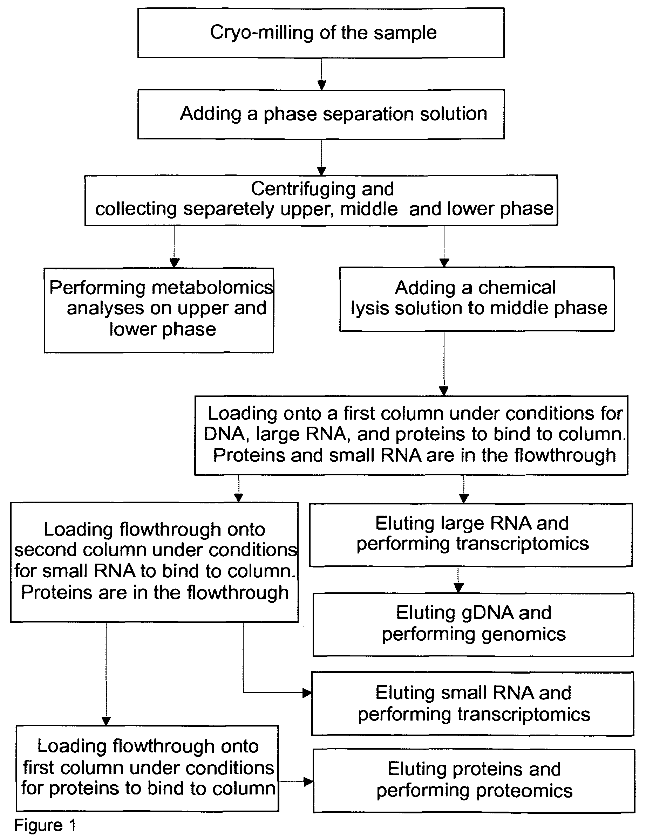

First reference should be taken to figure 1, representing the extraction method flowchart according to the invention. The sample is submitted to a cryogenic lysis step. With preference, this mechanical lysis is performed at at least -80°C in an oscillating mill at a frequency of about 30 Hz during at least 2 min. Cells, and for example, microbial cells are partially lysed by cryogenic grinding, with preference the cryo-milling step is performed in order that about 50 % of the cells are lysed indiscriminately.

Metabolites are first extracted in a phase separation step. A phase separation solution comprising a mixture of methanol, chloroform and water in the proportion of one volume of methanol, one volume of water and two volumes of chloroform is added to the cryo-milled sample. The ratio of chloroform (or other non-polar solvents considered) in the mixture can be lowered by the skilled man if the sample is not rich in lipid. Conversely, where the sample is expected to be rich in polar metabolites the ratio of methanol (or other polar solvent) can be higher. The sample mixed with the solvent mixture is homogenized and centrifuged. Polar and non-polar metabolites are extracted separately as they are solubilized either in the polar or in the non-polar phase. The centrifugation step allows a separation into three phases: an upper phase comprising polar metabolites, an interphase pellet comprising genomic DNA, large and small RNA, proteins and non-lysed cells, and a lower phase comprising non-polar metabolites. Metabolomic studies are performed on the lower and upper phase. The addition of the phase solution and the following homogenization step are performed at a temperature below 0°C in order to protect RNA and DNA.

A combined mechanical and chemical lysis step is performed on the interphase pellet which contains all remaining cellular constituents. The lysis solution comprises with preference Tris-EDTA and a lysis buffer. β-mercaptoethanol is added to the interphase together with the lysis solution to preserve RNA integrity. Following this, differential nucleic acid and protein isolation is carried out using chromatographic spin-columns.

In a first embodiment, the method involves for differential nucleic acid and protein isolation the commercially available "All-in-One Purification Kit" (Total RNA, microRNA, total proteins and genomic DNA) from Norgen Biotek Corp. Using this kit, the lysate is mixed with ethanol and is applied to a first mineral support (first column) under conditions for genomic DNA, large RNA and part of the proteins to bind. The flowthrough fraction which contains unbound proteins and small RNA is collected. The flowthrough is applied to a second mineral support (second column) under conditions for small RNA to bind; the flowtrough is again collected since it contains the proteins. Genomic DNA and large RNA are sequentially eluted from the first mineral support. The flowthrough containing proteins collected from the second mineral support is adjusted to pH 3 and is applied to the first mineral support under conditions to have the remaining proteins to bind together with the first bounded proteins. Then the proteins are eluted from the first mineral support. Transcriptomics is performed on the large and small RNA fractions. Genomics is performed on the genomic DNA fraction. The protein fraction is submitted to proteomics.

In another embodiment, the method involves for differential nucleic acid and protein isolation the commercially available AllPrep® DNA/RNA/Protein Mini kit (Qiagen, Valencia, CA). Using this kit, the lysate is passed through a QIAshredder column which allows selective binding of genomic DNA. Ethanol is then added to the flow-through to provide appropriate binding conditions for RNA onto the membrane of an RNeasy spin column. An aqueous protein precipitation solution is added to the flow-through for the isolation of intact total protein. The protein pellet is then re-dissolved in the designated buffer. RNA is eluded using a dedicated buffer.

In another embodiment, the method involves for differential nucleic acid and protein isolation known methods using chromatographic spin-columns allowing the isolation of a total RNA fraction comprising small RNA and large RNA. Additionally a small RNA fraction can be obtained separately by any known isolation procedure.

With preference, the first and second mineral support are porous or non-porous and comprised of metal oxides or mixed metal oxides, silica gel, silicon carbide resin, silica membrane, glass particle, powdered glass, quartz, Aluminia, Zeolite, Titanium Dioxide, or Zirconium Dioxide.

To bind RNA to the column, ethanol is added to the lysate or to the flow-through, however in an embodiment of the method ethanol is replaced with a dipolar atropic solvent selected from Acetone, Acetonitrile, Tetrahydrofuran (THF), Methyl Ethyl Ketone, N,N-Dimethylformamide (DMF) and Dimethyl Sulfoxide.

In another embodiment the lysis solution includes a chaotropic salt, non-ionic detergent (i.e. non-ionic surfactant) and reducing agent. With preference said chaotropic salt is Guanidine HCl. Preferably said non-ionic agent is selected from Triethyleneglycol Monolauryl Ether, (octylplhenoxy)Polyethoxyethanol, Sorbitari Monolaurate, T-octylphenoxyployethoxyethanol, or a combination thereof. Preferably the non-ionic detergent or combination thereof is in the range of 0,1-10 %. With preference the reducing agent is 2-Aminoethanethiol, tris-Carboxyethylphosphine (TCEP), or $\beta$-mercaptoethanol.

The invention now being generally described, it will be more readily understood by reference to the following examples, which are included merely for the purpose of illustration of certain aspects and embodiments of the invention, and are not intended to limit the invention.

Example 1: Sampling and biomass preparation, first lysis

Example 1-1

Lipid-rich sludge was sampled from the surface of the anoxic activated sludge basin of the Schifflange wastewater treatment plant (Luxembourg) on 4 October 2010, 25 October 2010, 13 December 2010, 25 January 2011 and 23 February 2011. Sampling was performed four times on different "islets" of sludge or areas of sludge "blankets" floating at the wastewater surface using a levy cane of 500 ml. Sludge samples were collected in 50 ml Falcon tubes then immediately snap-frozen in liquid nitrogen onsite. One sample was kept at 4°C for paraformaldehyde fixation of cells following SYBR® Green (Molecular Probes™, Invitrogen™) staining and biomass counting. The sludge biomass sampled on 13 December 2010 contained $5.89 \times 10^8$ (+/- $1.54 \times 10^8$) microorganisms per ml. Biomass subsampling was done first by crushing the frozen sludge with a sterile spatula on dry ice or in liquid nitrogen and, thus, maintaining the sample in frozen state. Five replicates of 200 mg of biomass from the same sampling tube were stored at -80°C until carrying out the extraction protocol for each replicate. The sample was then briefly thawed on ice followed by centrifugation at 4°C, 18,000 $x$ g for 10 min to separate the supernatant (~150 $\mu$l) from the biomass. The biomass fraction was immediately refrozen prior to homogenisation by cryo-milling for 2 min at 30Hz using stainless steel milling balls within a Retsch® Mixer Mill MM400 (Retsch, Haan, Germany). In contrast, the supernatant fraction immediately underwent metabolite extraction.

Example 1-2

Three fresh faecal samples, around 200 mg, were collected on 10 March 2011 from a young healthy individual and placed immediately on ice. From our previous results (not shown), in order to guarantee the integrity of the faecal RNA fraction, a one third dilution (weight/volume) of faecal samples had to be carried out using RNAlater (which preserves RNA) and the sample was homogenized by shaking for 5 min at 10 Hz by bead-beating with stainless steel milling balls. Faecal homogenate was obtained by suspension centrifugation at 700 x g for 1 min. Biomass pellets were obtained by centrifugation at 14,000 x g for 5 min. Three replicates were stored at -80 °C until homogenisation by cryo-milling for 2 min at 30Hz using stainless steel milling balls within a Retsch® Mixer Mill MM400.

Example 1-3

Fresh river water was collected from the Alzette River (Schifflange, Luxembourg) on 5 April 2011. Cells were concentrated from 40 l of surface water (collected from around 1 metre of depth) by tangential flow filtration, with a filtration area of 0.1 $m^2$ and molecular weight cut-offs of 10 kD at a flow rate of $\sim$1.5 l $x$ $min^{-1}$. The concentrated cells were pelleted by ultracentrifugation at 48,400 x g for 1 h at 4 °C. Each of three resulting pellets was diluted in 1 ml of supernatant. After SYBR® Green (Molecular Probes™, Invitrogen™) staining and biomass counting, we found that the concentrated biomass contained 1.48 $x$ $10^9$ (+/- 1.08 $x$ $10^7$) microorganisms per ml. Concentrated biomass pellets were obtained by performing an additional centrifugation step at 14,000 $x$ g for 5 min and stored at -80°C until biomolecular extraction.

Each of the three biomass samples were homogenised by cryo-milling for 2 min at 30 Hz using stainless steel milling balls within a Retsch® Mixer Mill MM400.

Example 2: Extracellular metabolite extraction

The extracellular metabolite isolation was performed only on supernatant from example 1-1. For example 1-2 and 1-3 supernatant separation was not possible because of the high biomass density encountered in the faecal samples and the need for concentrating the river water sample by tangential flow filtration which removes the supernatant.

The invention accommodates the possibility of an extracellular metabolite extraction step as demonstrated here on the lipid accumulating organism enriched sample. Extracellular metabolite extraction was performed on the supernatant collected following centrifugation of the sample. The extraction relies on prolonged soft mixing, with 150 $\mu$l of methanol, methanol/supernatant (1:1; v/v;) and 300 $\mu$l of chloroform, by vortexing during 10 min at 4°C. 6 mg/ml of ribitol solution is added in the polar phase as internal standard for the ensuing metabolomics experiment that allows assessment of minor variations that occur during the sample preparation and analysis steps.

Example 3: Intracellular metabolite extraction

The extraction was performed by mixing the biomass samples with a cold methanol/water/chloroform (1/1/2; v/v/v) solvent mixture. Ratio of chloroform is chosen to be double of the methanol because of the lipid rich nature of the samples. Intracellular metabolite extraction is performed with a mixture of 300 $\mu$l methanol/water (1:1; v/v) and 300 $\mu$l of chloroform and by bead-beating using the stainless steel balls (the same as used for the previous cryo-milling homogenization step) for 2 min at 20 Hz in a Retsch® Mixer Mill MM400. 6 mg/ml of ribitol solution was added in the polar phase as internal standard in order to assess the level of variation resulting from sample preparation and analysis.

After centrifugation at 14,000 $x$ g, 4°C for 10 min, a separation is observed among the polar top phase, interphase pellet and non-polar lower phase. The interphase pellet (along with the stainless steel milling balls) was kept at 4 °C for concomitant large RNA, genomic DNA, small RNA and protein isolation.

Example 4: Simultaneous large RNA, genomic DNA. small RNA and proteins isolation

The method steps according to the invention to concomitantly isolate large RNA, genomic DNA, small RNA and proteins was based on spin column chromatography involving the All-in-One Purification kit (NorgenBiotek Corporation, Thorold, ON) for which the protocol was modified.

The described procedure provides the possibility to separately isolate the large and the small RNA (< 200 nt) fractions. The cell pellet (biomass) was firstly lysed following mixing with Tris-EDTA (TE, 1X) and the NorgenBiotek lysis buffer (1/4; v/v) and by bead-beating in the buffer mixture using the stainless steel balls (the same as used for prior cryo-milling homogenization) for 30 sec at 20 Hz in a Retsch® Mixer Mill MM400 for immediate inactivation of DNases, RNases and proteases. beta-mercaptoethanol was also added in order to further prevent RNA degradation. The lysis buffer includes chaotropic agents that denature the lipids; therefore such agent is used, according to the invention, after the metabolic extraction.

The lysate was then mixed with 100 $\mu$L of pure ethanol (99 % ethanol) and was loaded onto a chromatographic spin column. In this step, large RNA, genomic DNA and a part of proteins were bound to the column while the small RNA (including microRNA) and other part of proteins were removed in the flow-through. It has to be noted that the addition of ethanol was carried out after the chemical and mechanical lysis of the cells and this mediates a highly efficient binding of the nucleic acids to the spin-column. The user can choose to separately isolate the large (> 200 nt) and the small/microRNA (< 200 nt) in modifying the amount of alcohol component (for example ethanol) added to the lysate. The bound large RNA and genomic DNA were then alternatively washed off the column and eluted two times with dedicated solutions. The flow-through was then loaded onto specific small RNA enrichment column allowing the purification of small RNA (including microRNA). The flow-through of the small RNA enrichment column was pH adjusted and loaded back onto the first column in order to bind the proteins that were present. The bound proteins were finally washed and eluted two times.

Example 5: Assessment of lysis efficiency as well as the quality and quantity of obtained biomolecular fractions

To assess the quantity and quality of obtained nucleic acids and proteins fractions as well as the lysis method according to the invention, the sequentially purified fractions and the cell lysis efficiency was compared to widely used reference methods, which were applied to the same samples following the cryomilling and metabolite extraction steps. Based on a brief literature review it was decided to compare the method to two different strategies for concomitant RNA/DNA/proteins extraction as well as respective dedicated and exclusive RNA, DNA and proteins extraction methods. Important to note that the extraction protocol according to the invention is the only process that allows small RNAs to be isolated separately. Concerning concomitant RNA/DNA/proteins extraction methods, the first one chosen was based on commercials available chromatographic spin columns methods, the AllPrep® DNA/RNA/Protein Mini kit (Qiagen, Valencia, CA, USA) and the second one was based on the TRI Reagent® (Sigma-Aldrich, Taufkirchen, Germany) a mixture composed of water, phenol and guanidine thiocyanate in a mono-phasic solution. These two methods were designed to purify and/or isolated genomic DNA, total RNA and total protein concomitantly from a single cell and tissue sample.

AllPrep® DNA/RNA/Protein Mini kit (Qiagen, Valencia, CA), integrates Qiagen's patented technology for selective binding of biomolecules on a silica-based membrane with the speed of microspin technology and combines this with protein precipitation chemistry. The procedure provides enrichment for mRNA since most RNAs < 200 nt, such as 5.8S rRNA, 5S rRNA, tRNA and miRNA are selectively excluded and, hence, these RNA fractions cannot be analysed.

TRI Reagent® (Sigma-Aldrich, Taufkirchen, Germany) is based on a highly selective liquid-phase separation where RNA, DNA and proteins are isolated respectively in the aqueous phase, interphase and organic phase (Chomczynski, P (1993) A reagent for the single-step simultaneous isolation of RNA, DNA and proteins from cell and tissue samples. Biotechniques 15:532-36, 236.) Briefly, the lysate is mixed with chloroform and centrifuged, which yields three fractionation phases. RNA is precipitated from the aqueous phase, by addition of isopropanol, washed and dissolved in RNase free water. DNA is precipitated from the interphase and organic phase by addition of ethanol, washed and dissolved in NaOH solution. Proteins are precipitated from the phenol-ethanol phase by the addition of isopropanol, washed and dissolved in urea-Tris-HCl/SDS 1% (1:1; v/v) (Hummon AB, Lim SR, Difilippantonio MJ, Ried T. (2007). Isolation and solubilization of proteins after TRIzol extraction of RNA and DNA from patient material following prolonged storage. Biotechniques 4:467-70, 472.)

For the dedicated exclusive biomolecular extraction widely used reference methods were chosen. DNA extraction was performed with the PowerSoil® DNA isolation kit (MO BIO laboratories, Carlsbad, CA). This method was used because it is a widely used method for isolating genomic DNA from environmental samples and results in DNA of high purity, allowing for PCR amplification and other downstream applications including random shotgun sequencing for genomics. The homogenization is performing by bead-beating on a vortex in supplied PowerBead tube. DNA purification was carried out using a chromatographic spin column, several wash steps and final elution using 100 μl of a 10 mM Tris buffer solution.

The RNA extraction was performed with RNeasy Mini kit (Qiagen, Valencia, CA). This kit was chosen because of its similarity with the RNA purification included in the AllPrep® DNA/RNA/Protein Mini kit (Qiagen, Valencia, CA; see above). The disruption and homogenization treatment uses a bead-beating cell disruption system in lysis buffers, followed by a selective passage through a membrane which binds RNA. A DNase treatment is performed at 30°C for 15 min to eliminate contaminating genomic DNA. The final RNA fraction is obtained by elution from the membrane in 100 μl of RNase-free water.

Proteins extraction was performed according to a metaproteomic extraction method developed on activated sludge (Wilmes P. and Bond PL. (2004). The application of two-dimensional polyacrylamide gel electrophoresis and downstream analyses to a mixed community of prokaryotic microorganisms. Environmental Microbiology 9:911-920) This method uses a wash step with a 0.9 % NaCl solution to remove excess exopolysaccharides, cell lysis is performed by French Press in combination with urea-thiourea-CHAPS buffers and protein purification by precipitation in 10 % (w/v) trichloroacetic acid and washes in 80 % (v/v) ice-cold acetone.

Example 5. 1: Assessment of cell lysis efficiency

The first step of any extraction process is cells lysis, where the cell membrane is disrupted, allowing the release of intact biomolecules. This crucial step determines the quality and quantity of the biomolecular fractions isolated downstream. In order to evaluate cell lysis efficiency and variation of each extraction protocol, a staining method able to differentiate lysed (dead) and non-lysed (viable) cells was used.

Before and after the lysis step, biomass was conserved at 4°C. A biomass pellet was obtained by centrifugation and washed with phosphate buffered saline solution (PBS, 1X) at pH 7 and observed by fluorescence microscopy following staining. Using this method, bacteria with intact cell membranes stain fluorescent green, and bacteria with damaged membrane stain fluorescent red. This method is known to the skilled man and is commercially available as Live/Dead® BacLight™ Bacterial Viability kit (Molecular Probes, Eugene, OR, USA). For determination of the lysis efficiency, the red fluorescence and green fluorescence micrographs were obtained and processed using the open-source image processing and analysis program ImageJ. A ratio was calculated between red and green mean pixel values in the micrographs and this value was subtracted by the same ratio calculated for the non-treated samples.

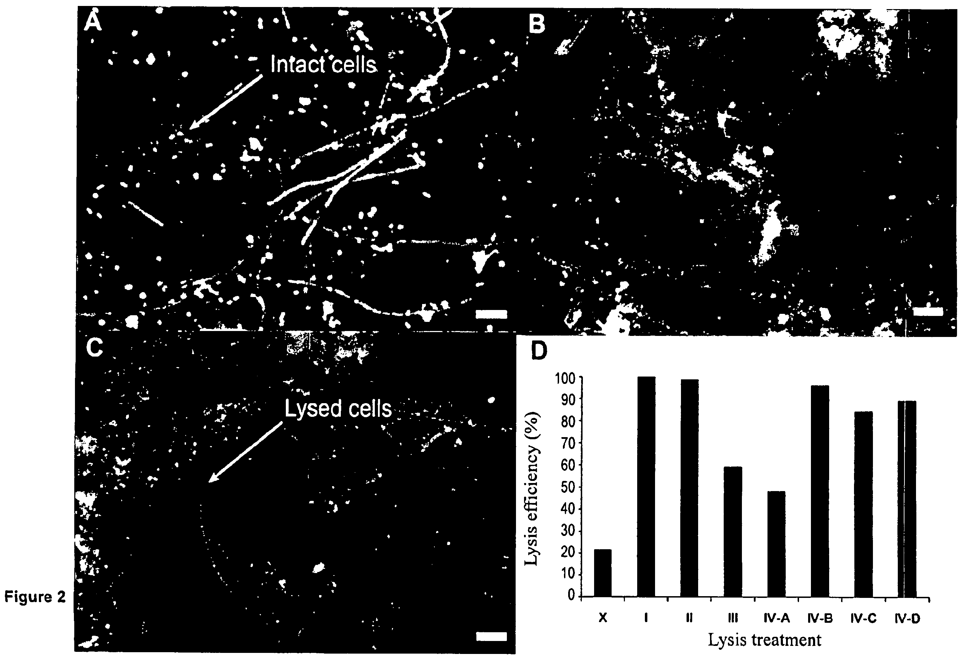

Figure 2 shows the lysis efficiency chart, representing the efficiency of different lysis methods. (A), (B) & (C) Representative fluorescence micrographs of microbial cells from lipid-rich biomass stained by the Live/Dead® BacLight™ Bacterial Viability kit. (A) Sample having undergone a single freeze-thaw cycle following sampling. (B) Sample having undergone additional metabolite extraction. (C) Sample having undergone the additional mechanical and chemical lysis step pertaining to the developed method using the NorgenBiotek All-in-One Purification kit's lysis buffer. In all panes, the scale bar is equivalent to $10\mu$m. (D) Barchart representing the lysis efficiencies, i.e. proportions of cells lysed, after different lysis methods. X: Sample having undergone a single freeze-thaw cycle, reflecting pane A. Treatments I-III: Sequential extraction protocols for DNA, RNA and proteins. I: NorgenBiotek All-in-One Purification kit's lysis buffer, (reflecting pane C). II: Qiagen AllPrep® DNA/RNA/Protein Mini kit's lysis buffer. III: TRI Reagent. IV: Cells stained after having undergone exclusive biomolecular extraction protocols for: IV-A, metabolite extraction, IV-B, DNA extraction. IV-C, RNA extraction, and IV-D, protein extraction. The non-treated sample (Figure 2A), which was submitted in example 1-1 to one freeze-thaw cycle following sampling, shows almost exclusively intact bacteria stained in green. Bacteria have thus retained their membranes despite the freeze-thawing cycle. The mechanical cryo-milling homogenisation treatment and metabolite extraction results in red lysis plaques representing lysed cells (Figure 2B). It can be observed that half of the cells preserved an intact membrane. After the second combined mechanical and chemical lysis step (Figure 2C), the lysed cells prevail. All micrographs demonstrate that the chosen lysis methods are comprehensive and indiscriminate of cell type.

As shown in Figure 2D, the lysis efficiency of the method according to the invention outperforms (albeit in some cases by a small margin) widely used reference methods for the concomitant or exclusive isolation of nucleic acids and proteins. These observations demonstrate the high efficiency of the lysis method pertaining to the invention which is essential for obtaining high-quality and representative biomolecular fractions.

Example 5.2: Quality control and measurement of method extraction efficiency

In order to perform quality control and measure the described method's extraction efficiency, biomolecular fractions were tested according to commonly used quantification and qualification methods. For this purpose, the extracted metabolites were analysed using gas chromatography coupled to mass spectrometry (GC/MS) analysis. The instrument used was an Agilent 7890A GC equipped with a 30m DB-35MS capillary column connected to an Agilent 5975C MS operating under electron impact (EI) ionization (Agilent Technologies Inc., Santa Clara, CA, USA).

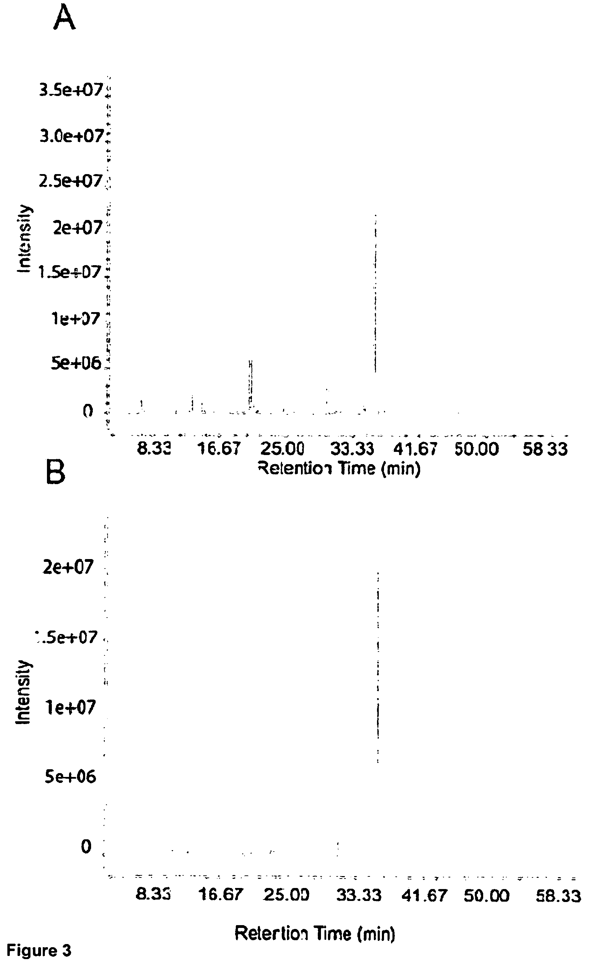

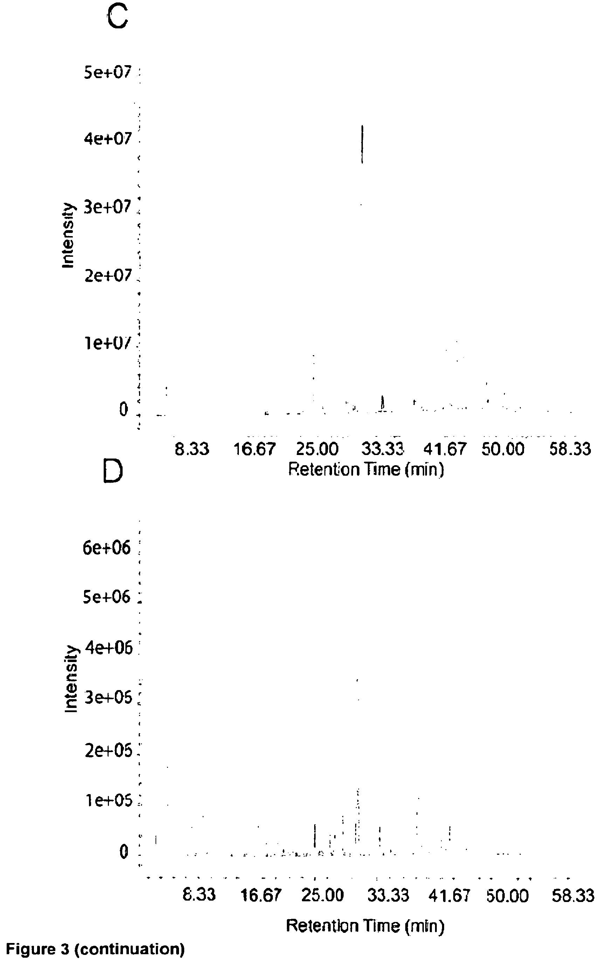

The resulting metabolomics data, i.e. total ion current (TIC) chromatograms (Figure 3), were interpreted by the use of the MetaboliteDetector software with a dedicated in-house library, which automatically carries out quantification of detected ions and performs an integration of ion peak intensities.

Spectrophotometric methods were used for measuring concentration and purity of nucleic acid and proteins fraction. Common electrophoresis analysis was used for molecular weight and integrity measurements. 2% agarose gel electrophoresis was used for the DNA fraction and sodium dodecyl sulphate polyacrylamide (SDS-PAGE) gel electrophoresis (Bio-Rad Laboratories, Hercules, CA) in conjunction with staining in LavaPurple protein stain (Fluorotechnics, Sydney, AUS) was employed for the protein fractions.

In addition to the SDS-PAGE separation of the obtained protein extracts, a particularly strong protein band was subjected to further quality assessment using a mass spectrometric bottom-up analysis. Briefly, after isolation and following digestion using the protease trypsin, the resulting peptides were spotted and subjected to matrix assisted laser desorption ionization time of flight tandem mass spectrometry (MALDI-ToF MS/MS) and high quality mass spectra were obtained demonstrating that high-quality protein fractions have been obtained (data not shown).

For RNA quality assessment we used an Agilent 2100 bioanalyser (Agilent Technologies, Santa Clara, CA). Two different kits, i.e. the Agilent RNA 6000 Nano kit and Agilent Small RNA kit for prokaryotes were used, for large and small RNA analysis, respectively. These kits allowed an assessment of the quantity and quality of the respective large (total) and small RNA fractions in addition to providing an assessement of critical parameters such as purity, yield and integrity of the RNAs.

In order to facilitate meaningful systems-level overviews of community- and population-wide biological processes by integration of high-resolution molecular data, one of the most important considerations is to obtain representative biomolecular fractions. Consequently, a particular attention was paid to quality assessment of obtained biomolecular fractions. The quality assessments were carried out using biomolecular extracts and fractions obtained using samples as described in example 1-1.

Figure 3 shows representative GC-MS total ion chromatograms of the metabolite fractions obtained from lipid accumulating organism biomass, with: (A) Intracellular polar metabolite fraction, (B) Extracellular polar metabolite fraction, (C) Intracellular non-polar metabolite fraction and (D) Extracellular non-polar metabolite fraction. Metabolomic analysis allowed the detection and quantification of 300 polar, 321 non-polar and 295 polar, 226 non-polar metabolite moelcules from the respective intra- and extra-cellular fractions (example 1-1). Reproducibility of the developed method was assessed by calculating the relative standard error of the intensity of the internal standard (ribitol) across the analysed samples. A relative standard error of 4.3 % was obtained. Consequently, the variability introduced into the analysis by instrument variation was small.

Nucleic acid fractions obtained using the different extraction protocols were firstly quantified by NanoDrop spectrophotometer, the 260:280 and the 260:230 ratios in particular reflecting purity of the respective fractions obtained. The median of 260:280 ratios for DNA fractions was between 1.9 and 2.1 and 2 for the developed method, generally accepted as "pure" and similar to those obtained with the other methods, the only exception being that the DNA extract obtained using the concomitant isolation of RNA/DNA/protein using the TRI reagent with which a poor ratio of 1.5 was measured instead the ratio >1.7 expected from the kit's product information. The large (total) and small RNA 260:280 ratios were found to be between 1.8 and 2.1 and 1.9 for the method according to the invention, indicating that overall high quality RNA was extracted by the different protocols.

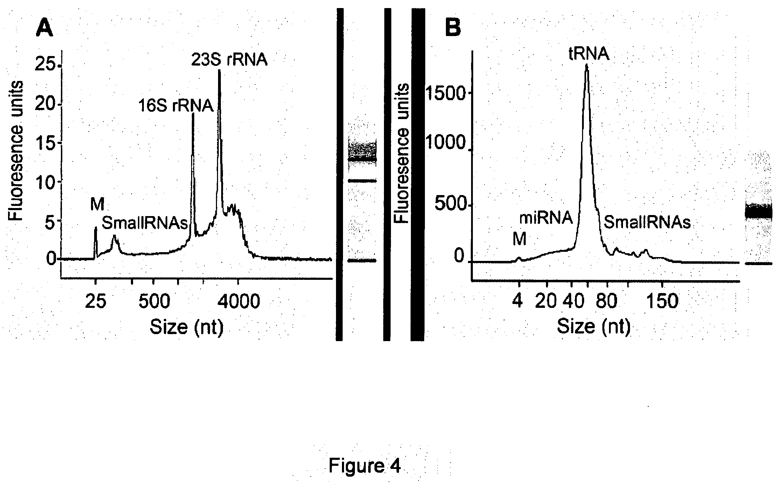

The quality of the respective RNA fractions (obtained from samples in example 1-1) were further analysed and verified using an Agilent 2100 Bioanalyser. The extraction method according to the invention isolated sequentially, among other biomolecular fractions, total RNA then small RNA. In case of degradation of total RNA, accumulation of small RNA fragments results in an overestimation of the miRNA and small RNA complement in samples. Therefore, it is critical to initially evaluate total RNA integrity. Integrity of the RNA samples was assessed using RNA Integrity Number (RIN) scores obtained from the Bioanalyser analysis. The mean RIN score of the total RNA fraction isolated the developed method was 7.0 (standard deviation of 1.20), indicating that high quality RNA was extracted. The score was quite similar to that obtained for exclusive RNA extraction (6.6, st. dev 0.88) and concomitant extraction based on the TRI Reagent method (7.4, st. dev 0.28) but lower than that obtained for the Qiagen concomitant RNA/DNA/proteins isolation method (9.7, st. dev. 0.00), which was a very high and a constant score. Figure 4 shows representative electropherograms of the RNA fractions obtained with the developed extraction method with (A) Large RNA fraction and (B) Small RNA fraction. (Abbreviations: M: marker, nt: nucleotides.) As shown on the electropherogram some DNA contamination may explain the score and this contamination can easily be removed using a subsequent DNase treatment (data not shown). Figure 4B highlights the major components of the small RNA fraction obtained with the developed method. Overall, the microRNA content of small RNA fraction (miRNA/smallRNA ratio) was high at 26.4 %.

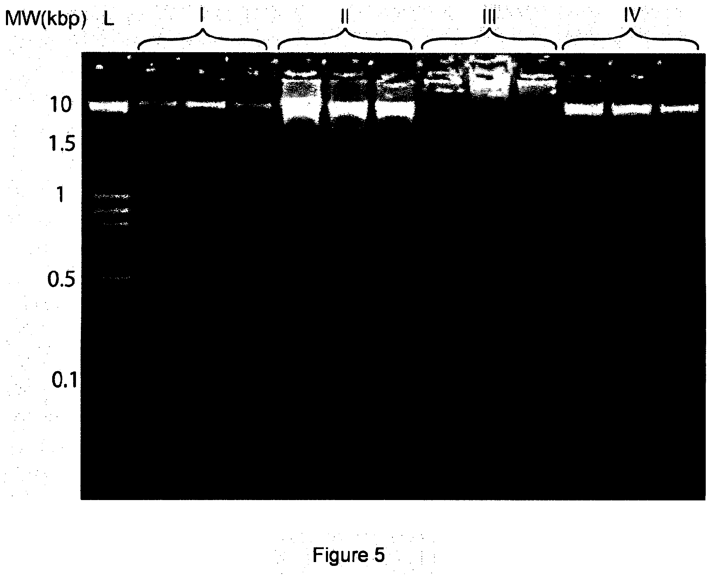

Figure 5 allows an appreciation of the size (kbp), the quality (degraded or intact) and semi-quantitative amount of DNA extracted. Figure 5 shows agarose gel electrophoresis gel image of the genomic DNA fractions extracted in triplicate by sequential (I, II, III) and exclusive (IV) extraction methods with lanes: (I): Developed method using the NorgenBiotek All-in-One Purification kit, (II): Developed method using the Qiagen AllPrep® DNA/RNA/Protein Mini kit, (III): Developed method using TRI Reagent phase separation, (IV): exclusive DNA extraction and (L): MassRuler™ DNA ladder mix. For a good quality of extracted genomic DNA, obtaining one distinct and thin band higher than 10 kbp without smearing at the bottom of the profile is expected. According to the results, rather expectably the exclusive standard extraction method presented the best DNA quality extract (Figure 5, lane IV). However, the genomic DNA fraction isolated by the developed extraction protocol (Figure 5, lane I) provided the most similar results to the reference method. Concerning the simultaneous extractions, the TRI reagent method (Figure 5, lane III) resulted in poor quality DNA extract with some very heavy DNA fragment but the majority being degraded and visible as smears on the gel. Simultaneous biomolecular isolation based on the Qiagen AllPrep® DNA/RNA/Protein Mini kit (Figure 5, lane II) exhibited intense and large bands with an important smears at the bottom. Importantly, the DNA fractions obtained using the extraction method according to the invention can be subjected immediately to polymerase chain reaction-based DNA amplification or random shotgun sequencing without the need for further purification (data not shown).

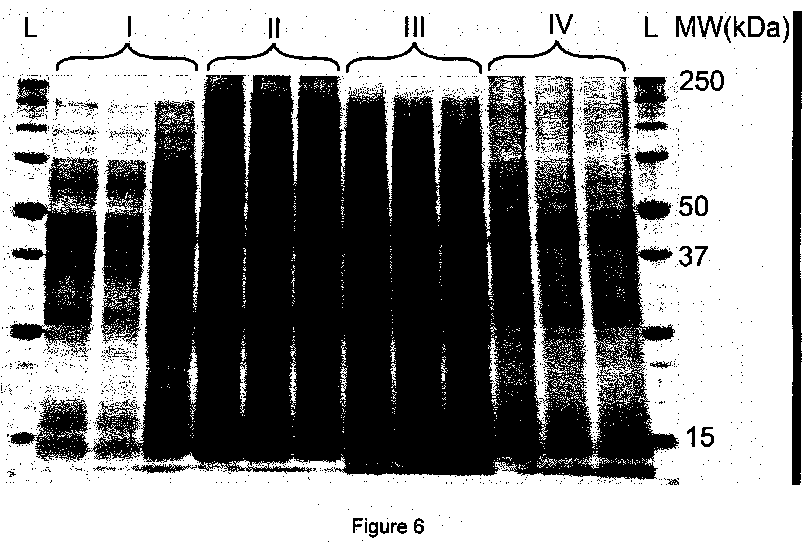

Figure 6 shows SDS-PAGE gel electrophoresis of protein fractions extracted in triplicate by sequential (I, II, III) and standard reference (IV) extraction methods, with lanes: (I): Developed method using the NorgenBiotek All-in-One Purification kit, (II): Developed method using the Qiagen AllPrep® DNA/RNA/Protein Mini kit, (III): Developed method using TRI Reagent phase separation, (IV): exclusive protein extraction method and (L): Precision Plus Protein™ Unstained standard ladder. The SDS-PAGE protein gel provides a visual representation of the community proteomes derived from the samples. A strong protein band is apparent at around 43 kDa in each sample. This band was subjected to bottom-up analysis and was putatively identified from peptide fragments (data not shown). Importantly, in terms of bands diversity and clarity, the efficiency of protein extraction was better for the simultaneous isolation extraction protocols (Figure 6, lanes I, II and III) than those obtained for the exclusive extraction method (Figure 6, lane IV). Many bands appeared distinctively on concomitant biomolecular extraction protein profiles (lanes I-III) and were less intense or missing for exclusive extraction method. This is due to removal of "contaminant" biomolecular fractions from the protein fraction during the concomitant biomolecular extraction methods whereas these biomolecular contaminants are retained in the extracts obtained with the exclusive extraction method. Consequently, the developed method results in more complete and representative protein extracts than the dedicated protein extraction method.

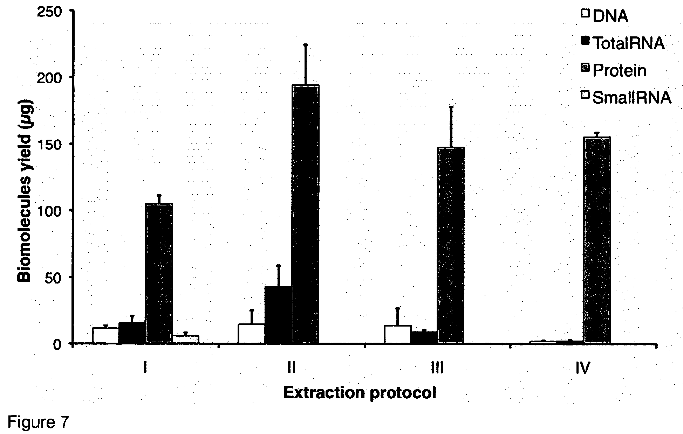

A quantitative assessment of extractions by measurement of yields obtained for each biomolecular fractions should be highlighted. Figure 7 shows a summary of yields obtains for each fraction for each extraction protocol with (I): Developed method using the NorgenBiotek All-in-One Purification kit, (II): Developed method using the Qiagen AllPrep® DNA/RNA/Protein Mini kit, (III): Developed method using the TRI Reagent phase separation and (IV): exclusive biomolecular extractions. The quantitative analysis was performed following the protocols specified above. For example, concerning the NorgenBiotek All-in-One Purification kit extraction method used in the invention, a second elution for genomic DNA and total RNA isolation was performed. For nucleic acids extraction, better yields were obtained for the concomitant extraction protocols than for the exclusive extraction protocols. However, the opposite was observed for protein extraction efficiency, where overall yields were better for exclusive isolation method. However, as discussed above the developed extraction method results in qualitatively superior protein extracts.

In terms of yields, the Qiagen AllPrep® DNA/RNA/Protein Mini kit (Figure 7) was the most efficient method for extracting simultaneously RNA, genomic DNA and proteins from a single sample with the best yield in terms of quantity and quality. However, the great advantage of our NorgenBiotek All-in-One Purification kit-based method is the ability to divide the extracted RNA into a large and small RNA fractions which can be then processed independently of each other.

Example 6: Sample heterogeneity determined by metabolomics.

The metabolome represents the output that results from the cellular interactions of the genome, transcriptome and proteome and, thus, should be the most sensitive indicator of cellular activity and, thus, sample-to-sample variation. Lipid-rich biomass, sampled at four different sludge areas and dates, were analysed to provide an assessment of microbial community sample variability which in turn provides an indication of the need for the invention to provide high-purity biomolecular fractions from a single biological sample.

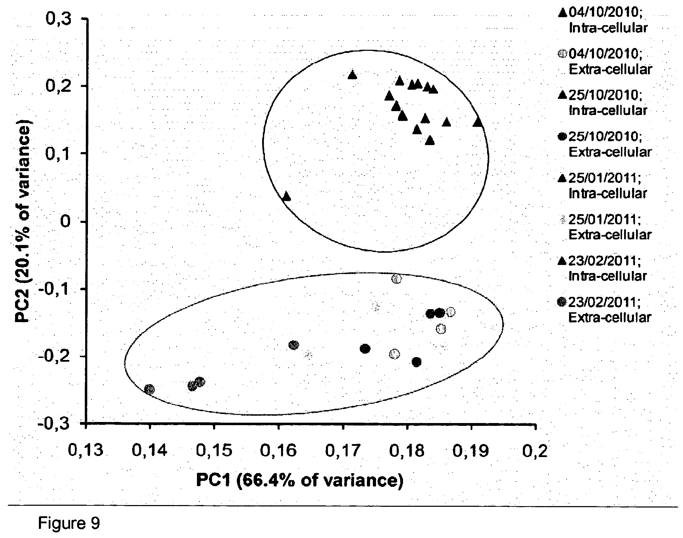

The raw GC-MS data were exported into a spreadsheet format using the MetabolicDetector software. Relative amounts of the various metabolites detected were obtained by unit vector normalizing the intensity of individual peaks. The matrix was then exported into the R statistical program for principal component analysis (PCA). Figure 9 shows principal component analysis (PCA) of combined polar and non-polar metabolomics data obtained from intra- and extra-cellular lipid accumulating organism-enriched biomass. For PCA, the combined polar and non-polar metabolite data was used, from both intra- and extra-cellular compartments, performed in quadruplicate biological replicates (different islets) and sampled at four different dates to appreciate the reproducibility of the sampling and the distinction between spatial (i.e. sampling points at the surface of activated sludge basin) and temporal (i.e. sampling dates) eco-systematic sample variation. PCA clearly distinguishes extra- from intracellular metabolomes. However, because of extensive sample variation, PCA is not able to discriminate between biological replicates of the same sampling date nor between the different sampling dates. This is a reflection of the extensive heterogeneity that is apparent within the sampled mixed microbial communities and highlights the need for the present invention in order to be able to discern meaningful linkages in the data following specialised omic analyses of the respective biomolecular fractions.

To assess the extent of temporal and spatial heterogeneity within mixed microbial communities, a comparative analysis of metabolome variability between replicates and sampling dates was performed. β-diversity analyses, traditionally used for comparing species diversity between eco-systems, was performed. This approach uses the Sørensen's similarity index (1) and the Bray-Curtis dissimilarity index (2).

$$\beta = \frac{2c}{S_1 + S_2}$$

(1)

β: Sørensen's similarity index, c: the number of metabolites common to both samples, $S_1$: the total number of metabolites recorded in the first sample, $S_2$: the total number of metabolites recorded in the second sample.

$$BC_{ij} = \sum \frac{|n_{ik} - n_{jk}|}{(n_{ik} + n_{jk})}$$

(2)

$BC_{ij}$: Bray-Curtis dissimilarity index, n: normalized intensity of individual peaks from GC/MS analysis of separate samples denoted i and j.

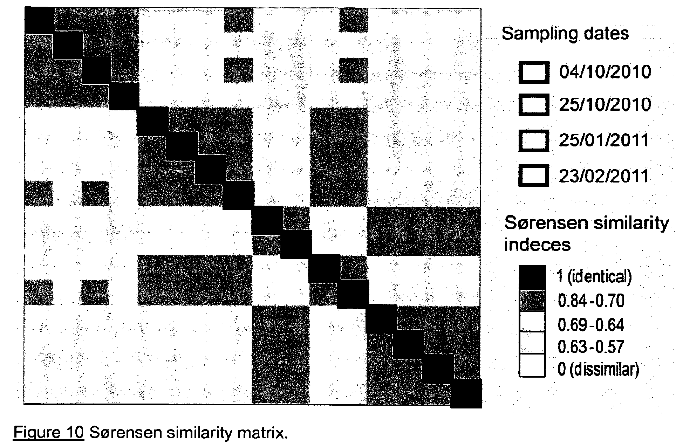

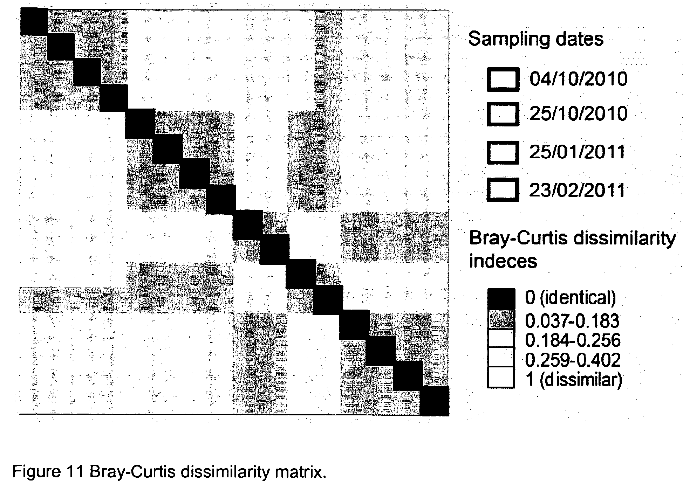

The analysis of the metabolomics data using both Sørensen similarity (Figure 10) and Bray-Curtis dissimilarity (Figure 11) indeces, again clearly highlight extensive variation between the samples which again reinforces the importance of the present invention. Figure 10 shows Sørensen's similarity matrix calculated based on intracellular polar metabolite data (most variable according to our analyses) derived from lipid accumulating organism-enriched biomass. Sampling dates: 04/10/2010, 25/10/2010, 25/01/2011 and 23/02/2011. Figure 11 shows Bray-Curtis dissimilarity matrix calculated based on intracellular polar metabolite data derived from lipid accumulating organism-enriched biomass. Sampling dates: 04/10/2010, 25/10/2010, 25/01/2011 and 23/02/2011.

Example 7: The universality of the method: human faeces and river water filtrate

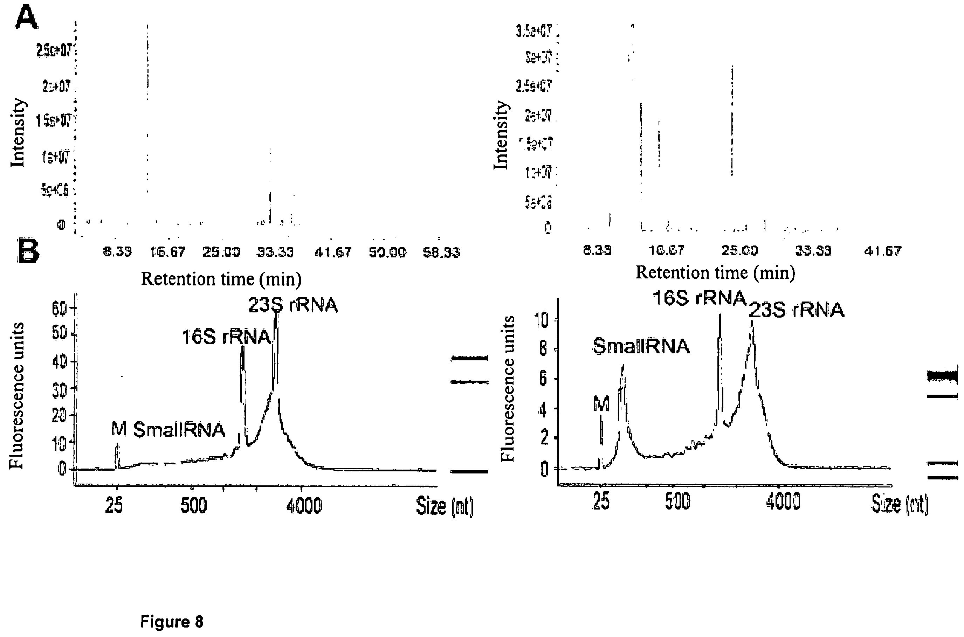

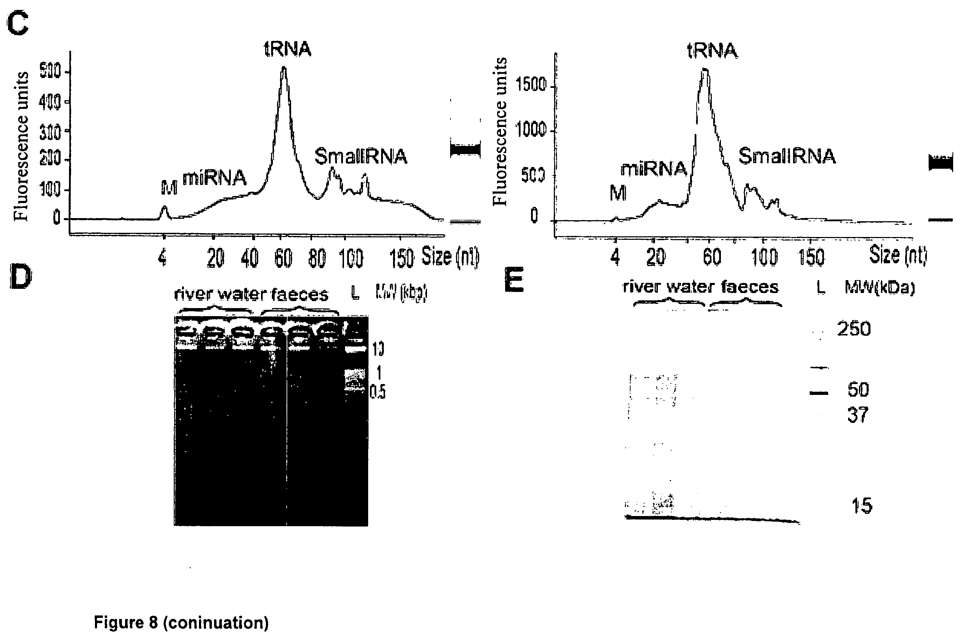

The developed biomolecular extraction method subject of the present invention allows the sequential isolation of polar and non-polar metabolites, genomic DNA, large and small RNAs fractions and proteins. As highlighted above, it was developed on lipid-rich biomass samples. The universality of the method was further tested by applying it to two additional mixed microbial community samples: human faeces and river water. Some minor sample-specific modifications were necessary as specified above. The analyses of the respective biomolecular fractions as presented in Figure 8, highlight comparable or even superior biomolecular fractions than those obtained from the lipid-rich biomass. Figure 8 shows biomolecular extractions carried out on different microbial community samples. The top three left-hand panes reflect river water filtrate extracts, top three right-hand panes reflect human faecal extracts with (A) Representative GC-MS total ion chromatograms of the polar metabolite fractions, (B) Representative electropherograms of the large RNA fractions, (C) Representative electropherograms of the small RNA fractions, (D) Agarose gel electrophoresis of the genomic DNA fractions, and (E) SDS-PAGE gel electrophoresis of protein fractions. Consequently, by applying the protocol to these two additional mixed microbial community samples we have proven that the method is applicable to a range of different biological samples.

**Claims**

1. A method for the separation and purification of cellular components from a single biological sample, the cellular components comprising polar and non-polar metabolites, genomic DNA, RNA and proteins, **characterized in that** the method encompasses the following steps:

   a) performing a mechanical lysis and homogenization of the single biological sample such that a part of the cells are lysed, the mechanical lysis being halted when 30 to 60 % of cells have been lysed;
   b) performing a metabolite extraction on the homogenized single biological sample from step (a) by addition of a phase separation solution, homogenization and centrifugation to form an upper phase, an interphase pellet and a lower phase; such that polar metabolites are in the upper phase, genomic DNA, RNA and proteins and the remaining cells not lysed by the mechanical lysis are in the interphase pellet, and non-polar metabolites are in the lower phase;
   c) collecting separately the upper phase, the lower phase and the interphase pellet;
   d) adding a lysis solution to the collected interphase pellet to perform a chemical lysis or a combined mechanical and chemical lysis, in order to obtain a lysate;
   e) performing a sequential isolation of genomic DNA, RNA and proteins on the lysate.

2. The method as claimed in claim 1, **characterized in that** the mechanical lysis of step (a) is halted when about 50 % of cells have been lysed.

3. The method as claimed in claim 1 or 2, **characterized in that** the mechanical lysis of step (a) is a cryo-milling step, with preference the cryo-milling step is performed at a temperature between -60° and -196°C.

4. The method as claimed in claim 3, **characterized in that** the cryo-milling step is performed in an oscillating mill at a frequency of 20 to 40 Hz, preferably 30 Hz, during about 2 min.

**5.** The method as claimed in claim 1 to 4, **characterized in that** the phase separation solution of step (b) comprises a mixture of methanol and chloroform and water in the proportion of 1 volume of methanol, 1 volume of water and two volumes of chloroform.

**6.** The method as claimed in any claims 1 to 5, **characterized in that** the addition of the phase separation solution of step (b) and the homogenizing of the sample is performed at a temperature below 0°C.

**7.** The method as claimed in any claims 1 to 6, **characterized in that** in step (d) the lysis solution comprises Tris-EDTA and a lysis buffer.

**8.** The method as claimed in any claims 1 to 7 **characterized in that** in step (d) β-mercaptoethanol is further added to the interphase to preserve RNA integrity.

**9.** The method as claimed in any claims 1 to 8 , **characterized in that** the biological sample is obtained with the steps of:

- collecting a sample and snap-freezing said sample directly after collection in liquid nitrogen, with preference at a temperature of -196°C;
- thawing the sample to a temperature comprised between 0°C and 4°C;
- centrifuging the sample to form a lower phase comprising biomass, and an upper phase comprising supernatant;
- collecting said biomass and freezing said biomass with preference at a temperature between -60°C and -196°C, with preference at a temperature of -196°C;
- using the frozen biomass as the single biological sample starting material for step (a).

**10.** The method as claimed in claim 9, **characterized in that** the supernatant is collected and submitted to a metabolite extraction in order to extract extracellular metabolites.

**11.** The method as claimed in claim 10 , **characterized in that** the metabolite extraction on the supernatant is performed with addition of a phase separation solution, homogenization and centrifugation of the mixture comprising the supernatant and the phase separation solution to form an upper phase, an interphase pellet and a lower phase; such that polar metabolites are in the upper phase, and non-polar metabolites are in the lower phase, and wherein with preference the phase separation solution consists of a mixture of methanol and chloroform and water in the proportion of 1 volume of methanol, 1 volume of supernatant and two volumes of chloroform.

**12.** The method as claimed in any of claims 1 to 11, **characterized in that** the sequential isolation of genomic DNA, RNA and proteins of step (e) comprises a step of isolation of small RNA from the single biological sample.

**13.** The method as claimed in any of claims 1 to 12, **characterized in that** the sequential isolation of genomic DNA, RNA and proteins of step (e) is carried out using chromatographic spin-columns.

**14.** The method as claimed in claim 13 **characterized in that** the sequential isolation of genomic DNA, RNA and proteins of step (e) further comprises the steps of

(e-1) Mixing lysate with dipolar atropic solvent or with polar tropic solvent such as ethanol to obtain a solution;
(e-2) Applying the solution of step (e-1) to a first chromatographic spin-column under conditions for genomic DNA, large RNA and part of the proteins to bind, and for obtaining a flowthrough;
(e-3) Collecting the flowthrough which contains small RNA and a part of the proteins;
(e-4) Applying the flowthrough of step (e-3) to a second chromatographic spin-column under conditions for small RNA to bind and for obtaining a flowthrough ;
(e-5) Eluting small RNA from the second chromatographic spin-column;
(e-6) Eluting sequentially genomic DNA and large RNA from the first chromatographic spin-column;
(e-7) Collecting the flowthrough of step (e-4) and adjusting the pH with preference to pH 3;
(e-8) Applying the pH adjusted flowthrough of step (e-4) to the first chromatographic spin-column;
(e-9) Eluting proteins from the first chromatographic spin-column.

**Patentansprüche**

**1.** Verfahren zur Trennung und Reinigung von Zellkomponenten aus einer einzelnen biologischen Probe, wobei die Zellkomponenten polare und nichtpolare Metaboliten, genomische DNA, RNA und Proteine umfassen, **dadurch gekennzeichnet, dass** das Verfahren die folgenden Schritte umfasst:

a) Durchführen einer mechanischen Lyse und Homogenisierung der einzelnen biologischen Probe derart, dass ein Teil der Zellen lysiert wird, wobei die mechanische Lyse gestoppt wird, wenn 30 bis 60% Zellen lysiert worden sind;

b) Durchführen einer Metabolitextraktion an der homogenisierten einzelnen biologischen Probe von Schritt (a) durch Zusatz einer Phasentrennungslösung, Homogenisierung und Zentrifugierung zur Bildung einer oberen Phase, eines Interphasenpellets und einer unteren Phase; sodass polare Metaboliten sich in der oberen Phase befinden, genomische DNA, RNA und Proteine und die von der mechanischen Lyse nicht lysierten restlichen Zellen sich in dem Interphasenpellet befinden, und nichtpolare Metaboliten sich in der unteren Phase befinden;

c) separates Auffangen der oberen Phase, der unteren Phase und des Interphasenpellets;

d) Zusetzen einer Lyse-Lösung zu dem aufgefangenen Interphasenpellet zwecks Durchführung einer chemischen Lyse oder einer kombinierten mechanischen und chemischen Lyse, um ein Lysat zu erhalten;

e) Durchführen einer sequentiellen Isolation von genomischer DNA, RNA und Proteinen an dem Lysat.

2.  Verfahren, wie in Anspruch 1 beansprucht, **dadurch gekennzeichnet, dass** die mechanische Lyse von Schritt (a) gestoppt wird, wenn ungefähr 50% Zellen lysiert worden sind.

3.  Verfahren, wie in Anspruch 1 oder 2 beansprucht, **dadurch gekennzeichnet, dass** die mechanische Lyse von Schritt (a) ein Kryo-Vermahlungsschritt ist; bevorzugt wird der Kryo-Vermahlungsschritt auf einer Temperatur zwischen -60° und -196°C durchgeführt.

4.  Verfahren, wie in Anspruch 3 beansprucht, **dadurch gekennzeichnet, dass** der Kryo-Vermahlungsschritt in einer Schwingmühle auf einer Frequenz von 20 bis 40 Hz, bevorzugt 30 Hz, während ungefähr 2 Minuten durchgeführt wird.

5.  Verfahren, wie in Anspruch 1 bis 4 beansprucht, **dadurch gekennzeichnet, dass** die Phasentrennungslösung von Schritt (b) ein Gemisch von Methanol und Chloroform und Wasser im Verhältnis von 1 Volumen Methanol, 1 Volumen Wasser und zwei Volumina Chloroform umfasst.

6.  Verfahren, wie in einem der Ansprüche 1 bis 5 beansprucht, **dadurch gekennzeichnet, dass** der Zusatz der Phasentrennungslösung von Schritt (b) und die Homogenisierung der Probe auf einer Temperatur unter 0°C durchgeführt wird.

7.  Verfahren, wie in einem der Ansprüche 1 bis 6 beansprucht, **dadurch gekennzeichnet, dass** in Schritt (d) die Lyse-Lösung Tris-EDTA und einen Lysepuffer umfasst.

8.  Verfahren, wie in einem der Ansprüche 1 bis 7 beansprucht, **dadurch gekennzeichnet, dass** in Schritt (d) der Interphase weiter β-Mercaptoethanol zugesetzt wird, um die RNA-Integrität zu bewahren.

9.  Verfahren, wie in einem der Ansprüche 1 bis 8 beansprucht, **dadurch gekennzeichnet, dass** die biologische Probe erhalten wird mit den Schritten des:

- Entnehmens einer Probe und Schockgefrierens dieser Probe direkt nach der Entnahme in flüssigem Stickstoff, bevorzugt auf einer Temperatur von - 196°C;

- Auftauens der Probe auf eine Temperatur zwischen 0°C und 4°C;

- Zentrifugierens der Probe zur Bildung einer unteren Phase, die Biomasse enthält, und einer oberen Phase, die Überstand enthält;

- Entnehmens der Biomasse und Gefrierens dieser Biomasse bevorzugt auf einer Temperatur zwischen -60°C und -196°C, bevorzugt auf einer Temperatur von -196°C;

- Verwendens der gefrorenen Biomasse als das einzelne biologische Proben-Ausgangsmaterial für Schritt (a).

10. Verfahren, wie in Anspruch 9 beansprucht, **dadurch gekennzeichnet, dass** der Überstand aufgefangen und einer Metabolitextraktion unterzogen wird, um extrazelluläre Metaboliten zu extrahieren.

11. Verfahren, wie in Anspruch 10 beansprucht, **dadurch gekennzeichnet, dass** die Metabolitextraktion an dem Überstand durchgeführt wird unter Zusatz einer Phasentrennungslösung, Homogenisierung und Zentrifugierung des den Überstand und die Phasentrennungslösung enthaltenden Gemischs, um eine obere Phase, ein Interphasenpellet und eine untere Phase zu bilden; sodass polare Metaboliten sich in der oberen Phase befinden und nichtpolare Metaboliten sich in der unteren Phase befinden, und wobei bevorzugt die Phasentrennungslösung aus einem Gemisch von Methanol und Chloroform und Wasser im Verhältnis von 1 Volumen Methanol, 1 Volumen Überstand und zwei Volumina Chloroform besteht.

**12.** Verfahren, wie in einem der Ansprüche 1 bis 11 beansprucht, **dadurch gekennzeichnet, dass** die sequentielle Isolation von genomischer DNA, RNA und Proteinen von Schritt (e) einen Schritt der Isolation von kleiner RNA aus der einzelnen biologischen Probe umfasst.

**13.** Verfahren, wie in einem der Ansprüche 1 bis 12 beansprucht, **dadurch gekennzeichnet, dass** die sequentielle Isolation von genomischer DNA, RNA und Proteinen von Schritt (e) unter Verwendung von Chromatographie-Spin-Säulen ausgeführt wird.

**14.** Verfahren, wie in Anspruch 13 beansprucht, **dadurch gekennzeichnet, dass** die sequentielle Isolation von genomischer DNA, RNA und Proteinen von Schritt (e) weiter die Schritte umfasst des

(e-1) Mischens von Lysat mit dipolarem aprotischem Lösungsmittel oder mit polarem protischem Lösungsmittel, wie etwa Ethanol, um eine Lösung zu erhalten;
(e-2) Einbringens der Lösung von Schritt (e-1) in eine erste Chromatographie-Spin-Säule unter solchen Bedingungen, dass genomische DNA, RNA und ein Teil der Proteine sich bindet und ein Durchfluss erhalten wird;
(e-3) Auffangens des Durchflusses, der kleine RNA und einen Teil der Proteine enthält;
(e-4) Einbringens des Durchflusses von Schritt (e-3) in eine zweite Chromatographie-Spin-Säule unter solchen Bedingungen, dass kleine RNA sich bindet und ein Durchfluss erhalten wird;
(e-5) Eluierens kleiner RNA aus der zweiten Chromatographie-Spin-Säule;
(e-6) sequentiell Eluierens genomischer DNA und großer RNA aus der ersten Chromatographie-Spin-Säule;
(e-7) Auffangens des Durchflusses von Schritt (e-4) und Einstellens des pH-Werts bevorzugt auf pH 3;
(e-8) Einbringens des pH-eingestellten Durchflusses von Schritt (e-4) in die erste Chromatographie-Spin-Säule;
(e-9) Eluierens von Proteinen aus der ersten Chromatographie-Spin-Säule.

## Revendications

**1.** Procédé pour la séparation et la purification de composants cellulaires à partir d'un échantillon biologique individuel, les composants cellulaires comprenant des métabolites polaires et apolaires, de l'ADN génomique, de l'ARN et des protéines, **caractérisé en ce que** le procédé comprend les étapes suivantes dans lesquelles :

a) on met en oeuvre une lyse mécanique et une homogénéisation de l'échantillon biologique individuel de manière telle qu'une partie des cellules est soumise à une lyse, la lyse mécanique étant stoppée lorsqu'on a obtenu une lyse des cellules à concurrence de 30 à 60 % ;
b) on procède à une extraction des métabolites à partir de l'échantillon biologique individuel homogénéisé que l'on obtient à l'étape (a) par addition d'une solution de séparation de phase, par homogénéisation et par centrifugation pour obtenir une phase supérieure, une pastille à l'interphase et une phase inférieure, de manière telle que les métabolites polaires se retrouvent dans la phase supérieure, que l'ADN génomique, l'ARN et les protéines ainsi que les cellules restantes non lysées par la lyse mécanique se retrouvent dans la pastille à l'interphase, et que les métabolites apolaires se retrouvent dans la phase inférieure ;
c) on récolte séparément la phase supérieure, la phase inférieure et la pastille à l'interphase ;
d) on ajoute une solution de lyse à la pastille récoltée à l'interphase pour mettre en oeuvre une lyse chimique ou bien une lyse mécanique et chimique combinée dans le but d'obtenir un lysat ;
e) on met en oeuvre une isolation séquentielle de l'ADN génomique, de l'ARN et des protéines sur le lysat.

**2.** Procédé selon la revendication 1, **caractérisé en ce que** la lyse mécanique de l'étape (a) est stoppée lorsqu'on a obtenu une lyse des cellules à concurrence d'environ 50 %.

**3.** Procédé selon la revendication 1 ou 2, **caractérisé en ce que** la lyse mécanique de l'étape (a) représente une étape de cryobroyage, l'étape de cryobroyage étant de préférence mise en oeuvre à une température entre -60 ° et -196 °C.

**4.** Procédé selon la revendication 3, **caractérisé en ce que** l'étape de cryobroyage est mise en oeuvre dans un broyeur oscillant à une fréquence de 20 à 40 Hz, de préférence de 30 Hz pendant environ 2 minutes.

5. Procédé selon les revendications 1 à 4, **caractérisé en ce que** la solution de séparation de phase de l'étape (b) comprend un mélange de méthanol et de chloroforme et d'eau dans la proportion de 1 volume de méthanol, 1 volume d'eau et 2 volumes de chloroforme.

6. Procédé selon l'une quelconque des revendications 1 à 5, **caractérisé en ce que** l'addition de la solution de séparation de phase de l'étape (b) et l'homogénéisation de l'échantillon sont mises en oeuvre à une température inférieure à 0 °C.

7. Procédé selon l'une quelconque des revendications 1 à 6, **caractérisé en ce que**, à l'étape (d), la solution de lyse comprend du Tris-EDTA et un tampon de lyse.

8. Procédé selon l'une quelconque des revendications 1 à 7, **caractérisé en ce que**, à l'étape (d), on ajoute en outre du β-mercaptoéthanol à l'interphase pour conserver l'intégrité de l'ARN.

9. Procédé selon l'une quelconque des revendications 1 à 8, **caractérisé en ce qu'**on obtient l'échantillon biologique en passant par les étapes consistant à :

- récolter un échantillon et soumettre ledit échantillon à une congélation rapide directement après sa récolte, dans de l'azote liquide, de préférence à une température de -196 °C ;
- décongeler l'échantillon jusqu'à une température comprise entre 0 °C et 4 °C ;
- centrifuger l'échantillon pour obtenir une phase inférieure comprenant une biomasse et une phase supérieure comprenant le produit surnageant ;
- récolter ladite biomasse et congeler ladite biomasse de préférence à une température entre -60 °C et -196 °C, de préférence à une température de -196 °C ;
- utiliser la biomasse congelée sous la forme d'un échantillon biologique individuel faisant office de matière de départ pour l'étape (a).

10. Procédé selon la revendication 9, **caractérisé en ce que** le produit surnageant est récolté et est soumis à une extraction des métabolites dans le but d'extraire les métabolites extracellulaires.

11. Procédé selon la revendication 10, **caractérisé en ce que** l'extraction des métabolites sur le produit surnageant est mise en oeuvre par addition d'une solution de séparation de phase, par homogénéisation et par centrifugation du mélange comprenant le produit surnageant et la solution de séparation de phase afin d'obtenir une phase supérieure, une pastille à l'interphase et une phase inférieure, de manière telle que les métabolites polaires se retrouvent dans la phase supérieure, et que les métabolites apolaires se retrouvent dans la phase inférieure, et dans lequel la solution de séparation de phase est constituée de préférence d'un mélange de méthanol et de chloroforme et d'eau dans la proportion de 1 volume de méthanol, 1 volume de produit surnageant et 2 volumes de chloroforme.

12. Procédé selon l'une quelconque des revendications 1 à 11, **caractérisé en ce que** l'isolation séquentielle de l'ADN génomique, de l'ARN et des protéines à l'étape (e) comprend une étape d'isolation du petit ARN à partir de l'échantillon biologique individuel.

13. Procédé selon l'une quelconque des revendications 1 à 12, **caractérisé en ce que** l'isolation séquentielle de l'ADN génomique, de l'ARN et des protéines à l'étape (e) est mise en oeuvre en utilisant des colonnes de centrifugation chromatographique.

14. Procédé selon la revendication 13, **caractérisé en ce que** l'isolation séquentielle de l'ADN génomique, de l'ARN et des protéines à l'étape (e) comprend en outre les étapes dans lesquelles :

(e-1) on mélange le lysat avec un solvant aprotique dipolaire ou avec un solvant protique polaire tel que l'éthanol pour obtenir une solution ;
(e-2) on applique la solution de l'étape (e-1) à une première colonne de centrifugation chromatographique dans des conditions qui permettent d'obtenir la liaison de l'ADN génomique, du grand ARN et d'une partie des protéines, et pour obtenir un écoulement continu ;
(e-3) on récolte l'écoulement continu qui contient le petit ARN et une partie des protéines ;

(e-4) on applique l'écoulement continu de l'étape (e-3) à une deuxième colonne de centrifugation chromatographique dans des conditions qui permettent d'obtenir la liaison du petit ARN et pour obtenir un écoulement continu ;

(e-5) on élue le petit ARN à partir de la deuxième colonne de centrifugation chromatographique ;

(e-6) on élue de manière séquentielle l'ADN génomique et le grand ARN à partir de la première colonne de centrifugation chromatographique ;

(e-7) on récolte l'écoulement continu de l'étape (e-4) et on règle le pH de préférence à un pH de 3 ;

(e-8) on applique l'écoulement continu de l'étape (e-4) dont le pH a été réglé, à la première colonne de centrifugation chromatographique ;

(e-9) on élue les protéines à partir de la première colonne de centrifugation chromatographique.

Figure 1

Figure 2

Figure 3

C

D

**Figure 3 (continuation)**

Figure 4

EP 2 751 268 B1

Figure 5

Figure 6

Figure 7

Figure 8

Figure 8 (coninuation)

EP 2 751 268 B1

Figure 9

Figure 10 Sørensen similarity matrix.

Figure 11 Bray-Curtis dissimilarity matrix.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7488579 B
- US 20050106604 A

- WO 2009070558 A

### Non-patent literature cited in the description

- **HUMMON AB ; LIM SR ; DIFILIPPANTONIO MJ ; RIED T.** Isolation and solubilization of proteins after TRIzol extraction of RNA and DNA from patient material following prolonged storage. *Biotechniques,* 2007, vol. 4, 467-70, 472
- **CHEY S ; CLAUS C ; LIEBERT UG.** Improved method for simultaneous isolation of proteins and nucleic acids. *Analytical Biochemistry,* 2011, vol. 1, 164-166
- **XIONG J ; YANG Q ; KANG J ; SUN Y ; ZHANG T ; MARGARET G et al.** Simultaneous isolation of DNA, RNA, and protein from Medicago truncatula L. *ELECTROPHORESIS,* 2011, vol. 2, 321-330
- **WECKWERTH W ; WENZEL K ; FIEHN O.** Process for the integrated extraction, identification and quantification of metabolites, proteins and RNA to reveal their co-regulation in biochemical networks. *PROTEOMICS,* 2004, vol. 1, 78-83
- **MORSE SM ; SHAW G ; LARNER SF.** Concurrent mRNA and protein extraction from the same experimental sample using a commercially available column-based RNA preparation kit. *Biotechniques,* 2006, vol. 1, 54, , 56, , 58

- **TOLOSA JM ; SCHJENKEN JE ; CIVITI TD ; CLIFTON VL ; SMITH R.** Column-based method to simultaneously extract DNA, RNA, and proteins from the same sample. *Biotechniques,* 2007, vol. 6, 799-804
- **GROSS V ; CARLSON G ; KWAN AT ; SMEJKAL G ; FREEMAN E ; IVANOV AR et al.** Tissue fractionation by hydrostatic pressure cycling technology: the unified sample preparation technique for systems biology studies. *J Biomol Tech,* 2008, vol. 3, 189-199
- **CHOMCZYNSKI, P.** A reagent for the single-step simultaneous isolation of RNA, DNA and proteins from cell and tissue samples. *Biotechniques,* 1993, vol. 15 (532-36), 236
- **HUMMON AB ; LIM SR ; DIFILIPPANTONIO MJ ; RIED T.** Isolation and solubilization of proteins after TRIzol extraction of RNA and DNA from patient material following prolonged storage. *Biotechniques,* 2007, vol. 4 (467-70), 472
- **WILMES P. ; BOND PL.** The application of two-dimensional polyacrylamide gel electrophoresis and downstream analyses to a mixed community of prokaryotic microorganisms. *Environmental Microbiology,* 2004, vol. 9, 911-920